# EUROPÄISCHE PATENTANMELDUNG

(11) **EP 0 987 026 A1**
(43) Veröffentlichungstag der Anmeldung: **22.03.2000**
(21) Anmeldenummer: 98810825.4
(22) Anmeldetag: 21.08.1998
(51) Int. Cl.: A61K 35/78

(54) **Verfahren zur Herstellung eines Kawa-Kawa-Extraktes**

(71) Anmelder: Max Zeller Söhne AG, 8590 Romanshorn (CH)
(72) Erfinder: Steiner, Rudolf, Prof. Dr.-Ing., 91056 Erlangen (DE); Tratz, Werner, 91052 Erlangen (DE); Prell, Werner, 91054 Erlangen (DE)
(74) Vertreter: Ritscher, Thomas, Dr.

(57) **Zusammenfassung**

Ein neuer Kawa-Kawa-Extrakt, der Kavapyrone als Wertstoffe und praktisch keine Flavokavine enthält, ist erhältlich durch ein einstufiges Verfahren zur Extraktion von Pflanzenmaterial von Piper methysticum sp., wobei man (a) das Pflanzenmaterial zur Gewinnung eines Direkt-Extraktes, der die Kavapyrone als Wertstoffe enthält, einer Hochdruckextraktion mit Kohlendioxid unterzieht, (b) die als Verunreinigungen vorhandenen Flavokavine im Zuge der Hochdruckextraktion durch Behandlung mit teilchenförmigem γ-Alumini-umoxid praktisch vollständig entfernt und (c) das Kohlendioxid vom Direkt-Extrakt trennt.

## Beschreibung

Die Erfindung betrifft ein Verfahren zur Herstellung einer neuen Form von Kawa-Kawa-Extrakt sowie den durch dieses Verfahren erhältlichen Extrakt. Hierbei wird als Kawa-Kawa-Extrakt ein solcher aus Pflanzenmaterial von Piper methysticum verstanden.

Solche Extrakte und Verfahren zu ihrer Herstellung sind in Form von Trocken- und Fluidextrakten z.B. aus EP 505 519 und 664 131 bekannt. Bei diesen bekannten Verfahren wird pulverisiertes Pflanzenmaterial, meist Rhizom von Piper methysticum Forst, in einer ersten Verfahrensstufe mit einem Lösungsmittel, wie Aceton, Chloroform, Ethyl-acetat oder einem Niederalkanol mit 1 bis 4 C-Atomen, der Wasser in Anteilen bis etwa 50 % enthalten kann, extrahiert und die Extraktlösung bis zur Trockenheit eingeengt.

Die Extraktion kann dabei durch Perkolieren oder durch eine Rühr- oder Wirbelextraktion bei normaler oder erhöhter Temperatur durchgeführt werden. In Abhängigkeit vom verwendeten Lösungsmittel enthalten die so erhaltenen und normalerweise tiefgelben Rohextrakte etwa 50 - 70 Gew.% Kavapyrone.

Dann wird der Rohextrakt in einer zweiten Verfahrensstufe erneut gelöst und einer Fällungsbehandlung durch Einwirkung von Kälte oder fällend wirkendem Nichtlösungsmittel unterzogen, z.B. unter Verwendung von mit Wasser mischbaren Lösungsmitteln oder durch Lösungsmittelverteilung zwischen einer wässrigen, wässrig-alkoholischen oder wässrig-acetonischen ersten Phase und einer mit dieser ersten Phase nicht mischbaren zweiten Phase. Die zweite Phase ist z.B. ein mit Wasser nicht mischbares organisches Lösungsmittel, wie Chloroform, Heptan, Hexan oder Ethylacetat.

Der so gewonnene gereinigte Extrakt enthält praktisch ebensoviel Kavapyrone (Wertstoffanteil) wie der Rohextrakt, aber weniger Flavokavine (unerwünschte Begleitstoffe, Farbstoffe), deren Anteil gegenüber dem Rohextrakt dadurch normalerweise um 60 - 80% reduziert wird.

Gemeinsam ist den bekannten Verfahren zur Herstellung von medizinisch akzeptablen Kawa-Kawa-Extrakten die Mehrstufigkeit, die nicht nur vergleichsweise zeit- und kostenaufwendig ist, sondern auch zu Extrakten führt, die sich erkennbar von einem Extrakt unterscheiden, der nach einem neuen einstufigen, d.h. direkten Verfahren gewonnen und hier als Direkt-Extrakt bezeichnet wird.

Gemäss Stand der Technik galt die Anwendung einer mindestens zwei Stufen umfassenden Arbeitsweise als Voraussetzung für die Gewinnung eines medizinisch akzeptablen Produktes, weil angenommen wurde, dass nur auf diese Weise ein ausreichend reiner Kawa-Kawa-Extrakt mit vermindertem Anteil an Flavokavinen gewonnen werden könnte.

Aufgabe der Erfindung ist es, einen Direkt-Extrakt zu bieten, wie er nur durch ein einstufiges Verfahren erhältlich aber dennoch praktisch frei von Flavokavinen ist.

Diese Aufgabe wird erfindungsgemäss dadurch gelöst, dass man (a) das Pflanzenmaterial zur Gewinnung eines neuen Direkt-Extraktes, der Kavapyrone als Wertstoffe enthält, einer Hochdruckextraktion mit Kohlendioxid unterzieht, (b) die als Verunreinigungen vorhandenen Flavokavine im Zuge der Hochdruckextraktion, d.h. in einstufigem Betrieb, durch Behandlung mit teilchenförmigem γ-Aluminiumoxid praktisch vollständig entfernt und (c) das Kohlendioxid vom Direkt-Extrakt trennt.

Der erfindungsgemäss erhältliche neue Direkt-Extrakt unterscheidet sich von den nach bekannten Verfahren erhaltenen Extrakten nicht nur durch die erfindungsgemäss ermöglichte Verbesserung der Wirtschaftlichkeit der Gewinnung hochreiner Extrakte, deren Gehalt an Flavokavinen bis an die Grenzen der Nachweisbarkeit und jedenfalls bis zu Flavokavin-Anteilen im Bereich von 10⁻⁹, d.h. in den ppb-Bereich) vermindert ist, sondern auch dadurch, dass ein praktisch farbloser Direkt-Extrakt erhalten wird, der sich beim Abkühlen auf Temperaturen von 20°C oder darunter unter Bildung kristalliner Bereiche oder Einschlüsse verfestigt.

Als Kavapyrone werden hier die bekannten substituierten δ-Lactone (Kavapyrone) mit einer β-ständigen Methoxygruppe im Ring, z.B. Kavain und dessen ebenfalls bekannte Derivate verstanden, wie z.B. das 7,8-Dihydrokavain, Methysticin, 7,8-Dihydromethysticin, Yangonin und 12-Desmethoxyyangonin gemäss der Formel 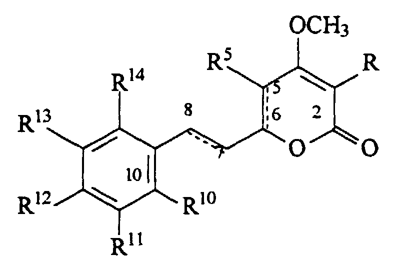 in der die 5-6- und 7-8- Bindungen Einfach- oder Doppelbindungen sind. Wenn alle R-Substituenten Wasserstoffatome sind, handelt es sich um Kavain, sofern die 5-6-Bindung eine Einfachbindung und die 7-8-Bindung eine Doppelbindung ist; wenn sowohl die 5-6- als auch die 7-8 Bindung Einfachbindungen sind, handelt es sich um Dihydrokavain. Yangonin unterscheidet sich von Kavain lediglich durch eine Methoxygruppe in Position 12, d.h. R¹² = CH₃O- , und durch eine Doppelbindung zwischen den Positionen 5 und 6. Das Desmethoxy-yangonin unterscheidet sich vom Yangonin durch die fehlende Methoxygruppe in Position 12. Im Fall von Methysticin bilden R¹² und R¹³ gemeinsam einen zweiwertigen Rest der Formel -OCH₂O-, während alle anderen R-Substituenten Wasserstoffatome sind und die 5-6-Bindung eine Einfachbindung ist; die 7-8-Bindung ist bei Methysticin eine Doppelbindung, bei Dihydromethysticin eine Einfachbindung. Spezielle Beispiele sind neben den eben genannten Verbindungen insbesondere, aber nicht abschliessend, die folgenden: 5,6,7,8-Tetrahydroyangonin, 7,8-Dihydro-5,6-dehydrokavain, 11,12-Dimethoxydihydrokavain, 7,8-Dihydro-5,6-dehydromesticin, 7,8-Dihydroyangonin, 5,6-Dehydromethysticin, 10- und 11-Methoxyyangonin, 5-Hydroxykavain, 11-Methoxy-12-hydroxy-dehydrokavain, usw..

Es wird angenommen, dass die Kavapyrone vor allem in ihrer Gesamtheit, d.h. mit dem in der Natur vorkommenden Spektrum einer Reihe von Verbindungen, Träger der pharmakologischen Wirksamkeit sind. So konnte gezeigt werden, dass die Bioverfügbarkeit von Kavapyronmischungen besser ist als die der Pyrone in reiner Form. Das pharmakologische Einsatzgebiet der aus Pflanzenmaterial erhältlichen Kavapyrone bzw. Kavapyronmischungen entspricht etwa dem der bekannten synthetischen Antidepressiva, wie z.B. die Benzodiazepine, und zwar ohne die Gefahr einer Abhängigkeit.

Als Flavokavine werden hier die bekannten Verbindungen der Formel 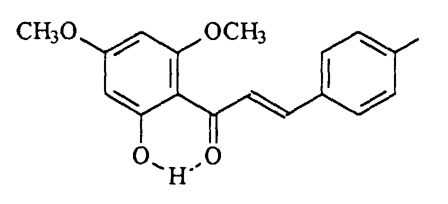 verstanden, in der R = H, OH oder OCH₃ darstellt.

Die Verwendung von verdichtetem Kohlendioxid zur Extraktion von Pflanzenstoffen einschliesslich Tabak, Kaffee und anderen pflanzlichen Wirkstoffen ist an sich bekannt. Es war jedoch nicht zu erwarten, dass die Anwendung auf die Herstellung von Kawa-Kawa-Extrakten besondere Vorteile bieten würde. Insbesondere war nicht zu erwarten, dass die kombinierte Anwendung von verdichtetem Kohlendioxid und γ-Aluminiumoxid zur Gewinnung eines medizinisch brauchbaren Kawa-Kawa-Extraktes führen würde, der trotz praktischer Abwesenheit der bei einstufigen Verfahren an sich zu erwartenden Anteile an verunreinigenden Flavokavinen als Direkt-Extrakt, d.h. nach einem einstufigen Verfahren erhältlich ist und den bekannten, nach zweistufigen Verfahren erhaltenen Kawa-Kawa-Extrakten mindestens gleichwertig, wenn nicht - weil vergleichsweise wertstoffhaltiger - überlegen ist.

Zur Hochdruckextraktion nach dem erfindungsgemässen Verfahren kann Kohlendioxid in flüssigem unterkritischem Zustand oder aber in überkritischem Zustand verwendet werden; typische bevorzugte Extraktionsbedingungen beim erfindungsgemässen Verfahren sind Drücke im Bereich von 100 - 400 bar und Temperaturen im Bereich von 20 - 80°C.

Im allgemeinen liegt die Dauer der Hochdruckextraktion zwischen etwa 10 und 60 Minuten, doch gilt keine dieser Grenzen als kritisch.

Das für die Extraktion verwendete Pflanzenmaterial hat zweckmässig eine Korngrösse von weniger als 500 µm, vorzugsweise eine solche im Bereich von 100 - 350 µm und insbesondere von 125 - 250µm.

Das zur Reinigung im Zuge der Extraktion verwendete γ-Aluminiumoxid (auch als Aktivtonerde, Merck, bezeichnet) hat vorzugsweise einen Wassergehalt von höchstens etwa 10 und vorzugsweise höchstens 5 Gew.%. Die Verwendung von technisch reinem γ-Aluminiumoxid wird bevorzugt, doch können grundsätzlich auch Mischungen aus Aluminiumoxiden verwendet werden, die in der Regel mindestens teilweise und vorzugsweise überwiegend oder praktisch Vollständig aus γ-Aluminiumoxid bestehen und weitere Aluminiumoxide, wie beispielsweise Bleicherde, enthalten, die ebenfalls wie γ-Aluminiumoxid im Unterschied zu den in dieser Technik üblichen Adsorptionsmitteln bei der Direkt-Extraktion eine praktisch vollständige Entfernung der Flavokavine ermöglicht, jedoch mit einer verminderten Ausbeute an Wertstoffen.

Gemäss einer bevorzugten Ausführungsform wird die Hochdruckextraktion mit einem Lösungsmittelstrom von mindestens etwa 2 kg Kohlendioxid/Stunde, bezogen auf 100 g Pflanzenmaterial, durchgeführt.

Zur Durchführung des erfindungsgemässen Verfahrens können an sich bekannte druckfeste Extraktionsbehälter verwendet werden, in welche das Kohlendioxid unter erhöhtem Druck eingeführt und von denen ein Strom von Direkt-Extrakt enthaltendem Kohlendioxid abgezogen werden kann; dabei wird der Strom zur Behandlung des extrakthaltigen Kohlendioxids durch mindestens eine Zone mit γ-Aluminiumoxid geführt, die im Extraktionsbehälter liegt oder/und diesem in einem separaten druckfesten Behälter nachgeschaltet ist.

Erfindungsgemäss ist ferner ein nach dem beschriebenen Verfahren hergestellter Direkt-Extrakt sowie ganz allgemein ein Kawa-Kawa-Extrakt, der Kavapyrone als Wertstoffe enthält und praktisch frei von Flavokavinen ist und der durch ein einstufiges Verfahren zur Extraktion von Pflanzenmaterial von Piper methysticum sp. erhältlich ist, wobei man (a) das Pflanzenmaterial zur Gewinnung eines Direkt-Extraktes, der die Kavapyrone als Wertstoffe enthält, einer Hochdruckextraktion mit Kohlendioxid unterzieht, (b) die als Verunreinigungen vorhandenen Flavokavine im Zuge der Hochdruck-extraktion durch Behandlung mit teilchenförmigem γ-Alumi-niumoxid praktisch vollständig entfernt und (c) das Kohlendioxid vom Direkt-Extrakt trennt.

In der beigeschlossenen Zeichnung zeigen:
Figur 1A einen schematischen Schnitt durch eine Vorrichtung zur Durchführung des erfindungsgemässen Verfahrens
Figur 1B einen Einsatz für die in Fig. 1 dargestellte Vorrichtung und
Figur 2 ein Fliessbild einer bevorzugten Ausführungsfoprm des erfindungsgemässen Verfahrens

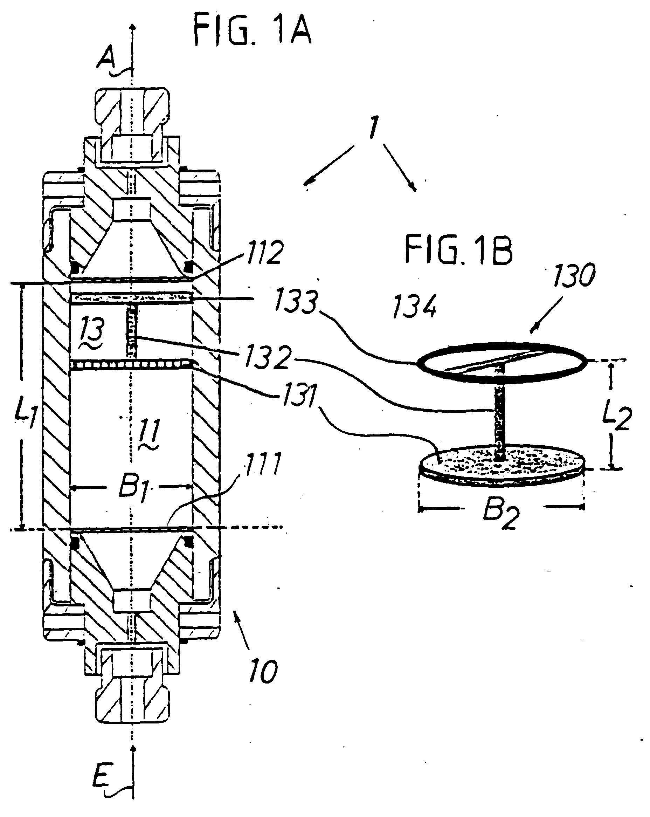

Im einzelnen zeigt Fig. 1A in schematischem Längsschnitt die Vorrichtung 1 mit einem Druckbehälter 10, der durch eine (nicht dargestellte) Zuleitung in Richtung des Pfeils E mit flüssigem oder überkritischem Kohlendioxid versorgt wird, das durch einen Siebboden 111 in den mit (nicht dargestelltem) Extraktionsgut gefüllten Extraktionsraum 11 gelangt und zusammen mit dem extrahierten Material durch eine Sieb- oder grobe Frittenplatte 131 in den mit teilchenförmigem □-Aluminiumoxid gefüllten Adsorptionsraum 13 und aus diesem durch eine weitere Siebplatte 112 in Richtung des Pfeils A durch eine (nicht dargestellte) Leitung abgezogen wird.

Das so gewonnene Produkt besteht aus Direkt-Extrakt und Kohlendioxid und kann in an sich bekannter Weise durch Expandieren zur Gewinnung des fertigen Direkt-Extrakts und eines Kohlendioxidstroms zerlegt werden. Der Kohlendioxid-Strom kann durch Komprimieren wieder verflüssigt bzw. in überkritischen Zustand umgewandelt und erneut in Richtung des Pfeils E in den Hochdruckbehälter 10 eingespeist werden.

Der in Fig. 1B dargestellte Einsatz 130 besteht aus der Sieb- oder Frittenplatte 131, die mittels eines Stabes 132 am Steg 134 befestigt ist. Der Steg 134 ist seinerseits mit dem Haltering 133 verbunden, der durch (nicht dargestellte) Nasen an der Innenwand 110 des Hochdruckbehälters 10 in Arbeitsstellung gehalten wird.

Der Druckbehälter kann in an sich bekannter Weise mit Einrichtungen zum Beschicken und Entladen des Extraktionsraums 11 und zum Beschicken und Entladen des Adsorberraums 13 versehen sein. Um einen kontinuierlichen Extraktionsbetrieb zu ermöglichen, können zwei oder mehrere Hochdruckbehälter 10 parallel geschaltet und alternierend in Betrieb genommen werden. Der Extraktstrom aus Kohlendioxid und Direkt-Extrakt kann mehrmals ohne Zerlegung rezirkuliert werden, doch wird dies normalerweise weniger bevorzugt, weil es jedenfalls für einen kommerziellen Betrieb zweckmässiger ist, das Extraktionsgut mit reinem bzw. nach Trennung vom Direkt-Extrakt zurückgewonnenem Kohlendioxid zu extrahieren.

Dank der hohen Wirksamkeit des erfindungsgemäss als Adsorbens zur Entfernung der Flavokavine verwendeten □-Aluminiumoxyds kann das Verhältnis des Extraktionsraum-Volumens zum Adsorptionsraum-Volumen verhältnismässig gross gehalten werden, z.B. 2:1 oder vorzugsweise mehr, d.h. mindestens 3:1. Bezogen auf die Gesamtlänge L1 des zylindrischen Innenraums des Druckbehälters 10 von Fig. 1A beträgt das Verhältnis der im wesentlichen durch den Abstand L2 bedingten Länge des Adsorptionsraumes 13 zur Gesamtlänge L1 vorzugsweise mindestens etwa 1:3.

Zur Durchführung des erfindungsgemässen Verfahrens können kommerziell erhältliche Druckbehälter verwendet werden, die lediglich durch die in Fig. 1B dargestellte Halterung für das Adsorbens modifiziert sind. Im übrigen ist es für das erfindungsgemässe Verfahren keineswegs erforderlich, dass die Behandlung des Extraktionsstromes mit dem Adsorptionsmittel im gleichen Behälter wie die Extraktion erfolgt. Vielmehr kann es für den technischen Betrieb vorteilhaft sein, die Behandlung mit dem Adsorbens in einem separaten Druckbehälter durchzuführen, der dem Extraktionsbehälter bzw. mehreren parallel arbeitenden Extraktionsbehältern nachgeschaltet ist.

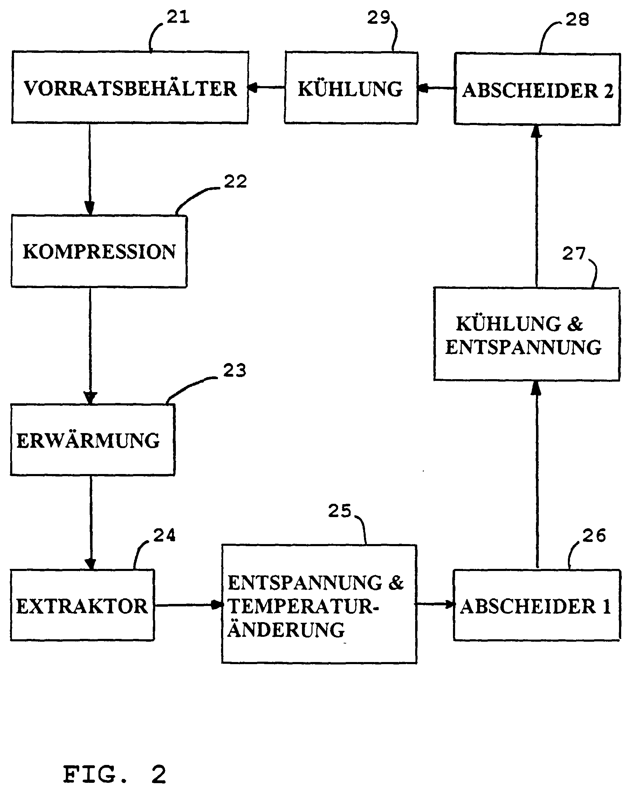

Der in Fig. 1A dargestellte Extraktor wird vorzuhsweise nach einem Verfahren hergestellt, dessen Fliessbild in Fig. 2 dargestellt ist. Das aus dem Vorratsbehälter 21 entnommene Kohlendioxid wird bei 22 auf Extraktionsdruck (je nach Verwendung von flüssigem bzw. überkritischem CO₂ für die Extraktion) komprimiert, gegebenenfalls bei 23 erwärmt und durch den gegebenenfalls beheizten Extraktor 24 geführt, wo es unter den gewählten Bedingungen mit Extraktstoffen beladen wird. Der Extraktstrom aus den Extraktstoffen und dem CO₂ wird nun bei 25 einer ersten Fraktionierung zur Abscheidung der Extraktstoffe durch eine erste Dekompression und gegebenenfalls Temperaturänderung unterzogen; im ersten Abscheider 26 werden dann praktisch alle Extraktionskomponenten abgeschieden. Nach einer zweiten Dekompression und gegebenenfalls Kühlung bei 27 gelangt der Extraktionsstrom in einen zweiten Abscheider 28 zur Abtrennung von Wasser und allenfalls darin gelösten Extraktstoffen. Das bei 28 abströmende praktisch reine Kohlendioxid gelangt nach Kühlung zur Verflüssigung bei 29 wieder in den Vorratsbehälter 21, aus dem es erneut für die Extraktion verwendet werden kann. Es versteht sich, dass das in Fig.2 dargestellte Fliesschema weder die zum Betrieb nötigen Steuerungs- noch Fördereinrichtungen zeigt, da diese den Fachleuten geläufig sind.

Das erfindungsgemässe Verfahren wird nun lediglich zum besseren Verständnis und nicht zur Beschränkung an Hand von Beispielen erläutert. Sofern nicht anders angegeben, sind Angaben in Teilen bzw. Prozenten auf das Gewicht bezogen.

Zur Extraktion wurde in allen Beispielen ein Extraktor mit dem in Fig. 1A dargestellten Aufbau verwendet (L₁=318mm; B₁=40mm), der für einen Maximaldruck von 1000-bar bei 100°C ausgelegt und mit einer Heizung versehen war. Der Extraktor wurde vor Versuchsbeginn mit dem Extraktionsgut beschickt und dann in einer Laboranlage mit dem in Fig. 2 gezeigten Aufbau betrieben. Das Kohlendioxid hatte eine Reinheit von mindestens 99,5% und einen maximalen Wassergehalt von 60 ppm.

Das Extraktionsgut bestand aus handelsüblichem zerkleinertem Wurzelstock von Kava-Kava (Dixa AG, St. Gallen, Schweiz) mit einem Wassergehalt von ca 8 Gew.%. Es wurden Chargen mit unterschiedlicher Korngösse und Korngrössenverteilung verwendet (Charge 1: durchschnittliche Korngrösse 170 µm, Krongrössenbereich 100 - 400 µm. Charge 2: maximale Korngrösse 180 µm; Charge 3: maximale Korngrösse 250 µm). Der Gehalt der Extrakte an Wertstoffen (Kavapyrone) und Verunreingungen (Flavokavine) wurde mittels Hochleistungs-Flüssigchromatografie und Gas-Chromatografie bestimmt.

### Beispiel 1

In diesem Beispiel wurde der Extraktor mit ca. 170 g Kava-Kava Rhizom (Charge 1) beschickt und bei 20 °C mit einem Lösungsmittelstrom von 4000 g CO₂/Stunde extrahiert. Der Extraktionsdruck wurde zwischen 70 und 350 bar variiert, d.h. das Kohlendioxid befand sich in flüssigem (unterkritischem) Zustand. Die Fraktionierung im ersten Abscheider 26 erfolgte bei 60 bar/50°C, die im zweiten Abscheider 28 bei 50 bar/20 °C. Die Extraktausbeute betrug 4 - 4,5 %; dieser Wert wurde bei 350 und 250 bar bereits innerhalb von etwa 30 Minuten erreicht, bei 100 bzw. 150 bar erst nach etwa 60 Minuten und bei 70 bar nach etwa 140 Minuten.

Bei einer zum Vergleich durchgeführten Versuchsreihe wurde der Einsatz 130 (Fig. 1B) entweder nicht mit Adsorbens oder mit nicht erfindungsgemässem Adsorbens (Aktivkohle, Ionenaustauscherharze: Amberlite®, Lewatit® und Baylith®, Polyamid, Polyvinylpyrrolidon und Polyvinyl-polypyrrolidon, Füllhöhe in der Adsorbenskammer 13 gemäss Fig. 1A jeweils ca. 90 mm) beschickt. Dabei wurde gefunden, dass der Flavokavin-Gehalt des Extraktes ohne Verwendung von Adsorbens Werte im Bereich von etwa 0,9 bis 1,5 % erreichte; mit Ionenaustauscherharz lagen die Flavokavinwerte im Bereich von 1,1 - 1,5% und nur bei Polyamid und Polyvinylpyrrolidon unter 1%. Die trockenen Extrakte waren gelb-rot bis rötlich-braun und hatten eine wachsartig zähe und schmierige Konsistenz.

Bei Verwendung von γ-Aluminiunoxid und/oder Bleicherde konnte bei gleicher Füllhöhe im Extrakt kein Flavokavin festgestellt werden. Der trockene Kava-Kava-Extrakt war praktisch weiss und zeigte kristalline Anteile. Im Fall von γ-Aluminiumoxid war die Wertstoffausbeute, bezogen auf das Gewicht des extrahierten Pflanzenmaterials, praktisch doppelt so hoch (4,1%) wie bei Verwendung von Bleicherde (2,1%). Die Selektivität von γ-Aluminiumoxid ist somit in überraschender Weise besser als diejenige der chemisch ähnlichen Bleicherde. Bei Verwendung von Aktivkohle wurde praktisch der gesamte Wertstoffanteil sowie Flavokavin vom Adsorbens aufgenommen und zurückgehalten. Aktivkohle als Adsorbens zeigt trotz des ausgeprägten Farbstoffcharakters der Flavokavine überraschenderweise praktisch keine Selektivität.

### Beispiel 2

Nach der in Beispiel 1 angegebenen Arbeitsweise wurden analoge Extraktionsversuche, jedoch mit überkritischem Kohlendioxid (150, 250 und 350 bar; 40, 55 und 70°C), durchgeführt. Dabei wurden ähnliche Ergebnisse wie in Beispiel 1 erzielt und die überraschend vorteilhafte Wirkung (quantitative Entfernung von Flavokavin bei sehr guter Wertstoffausbeute) von γ-Aluminiumoxd bei der Direkt-Extraktion von Kava-Kava Pflanzenmaterial festgestellt. Die Temperaturerhöhung führte zur einer etwa 10 - 30 %igen Zunahme der Wertstoffausbeute und zu einer rascheren Extraktion. Die besten Ausbeuten und Extraktionszeiten wurden bei Drücken im Bereich von 250 - 350 bar und Temperaturen im Bereich von 70- 50 °C erzielt.

Im Unterschied zu Beispiel 1 wurde Wasser in vermehrtem Anteil durch das überkritische CO₂ extrahiert und bei Dekompression abgeschieden. Daher wurde der Druck im ersten Abscheider 26 konstant auf 60 bar und die Temperatur unter Anpassung an die Extraktionstemperatur von 50 auf bis zu 80°C erhöht, um die Löslichkeitsgrenze des Wassers in CO₂ im Abscheider 26 nicht zu unterschreiten.

### Beispiel 3

In einer weiteren Versuchsreihe nach der Arbeitsweise von Beispiel 2 wurde der Einfluss der Korngrösse des Pflanzenmaterials auf Extraktionsdauer und -ausbeute untersucht. Dabei wurde gefunden, dass Korngrössen im Bereich von 355 - 1400 µm zu signifikant geringeren Ausbeuten führen. Der Korngrössenbereich im Bereich von 150 - 250 µm erwies sich als insgesamt am günstigsten.

### Beispiel 4

Zur Untersuchung der Wirkung des (im wesentlichen vom Wassergehalt abhängigen) Aktivitätsgrades von γ-Aluminiumoxid wurde eine weitere Versuchsreihe durchgeführt und dazu der Wassergehalt des γ-Aluminumoxides zwischen 0 und 20 % variiert. Bei einem Lösungsmittelverhältnis von 30,8 Liter/Stunde, einem Extraktionsdruck von 350 bar und einer Extraktionstemperatur von 55 °C wurde gefunden, dass optimale Ergebnisse bei einem Wassergehalt des als Adsorbens verwendeten γ-Aluminiumoxides im Bereich von 0 - 5 % erhalten werden. Die mit abnehmendem Wassergehalt zunehmende Verfärbung des Adsorbens entspricht diesem Befund.

### Beispiel 5

In einer weiteren Versuchsreihe wurde der Einfluss des Massenverhältnisses zwischen Adsorptionsmittel und Extraktionsgut untersucht. Dazu wurde nach der Arbeitsweise von Beispiel 2 bei einem Lösungsmittelverhältnis von 30,8 Liter/Stunde, einem Extraktionsdruck von 350 bar und einer Extraktionstemperatur von 50 - 60 °C das Massenverhältnis von γ-Aluminiumoxid (mit 5% Wassergehalt) zu trockenem Pflanzenmaterial wie folgt gewählt: 1:8, 4:15, 3:7 und 8:13. Die höheren Verhältniswerte (3:7 bzw. 8:13) erwiesen sich als zur Entfernung von Flavokavinen im Direkt-Extraktionsverfahren am besten geeignet.

Zusammenfassend bietet die Erfindung einen neuen Kawa-Kawa-Extrakt, der Kavapyrone als Wertstoffe aber praktisch keine Flavokavine enthält und durch ein einstufiges Verfahren zur Extraktion von Pflanzenmaterial von Piper methysticum sp., erhältlich ist, bei dem man das Pflanzenmaterial zur Gewinnung eines Direkt-Extraktes, der die Kavapyrone als Wertstoffe enthält, einer Hochdruckextraktion mit Kohlendioxid unterzieht und die Flavokavine noch im Zuge der Hochdruckextraktion, d.h. ohne vorherige Isolation des Primärextraktes, durch Behandlung mit teilchenförmigem γ-Aluminiumoxid vollständig entfernt und das Kohlendioxid vom Direkt-Extrakt trennt. Ein solches Herstellungsverfahren ist den bekannten Verfahren nicht nur durch die erheblich vereinfachte und daher wirtschaftlich vorteilhaftere Produktionsmöglichkeit sondern auch dadurch überlegen, dass der Extrakt so rein ist, dass er zur Bildung eines praktisch farblosen und mindestens teilweise kristallinen Produktes befähigt ist.

## Patentansprüche

1. Verfahren zur Herstellung eines Extraktes aus Pflanzenmaterial von Piper methysticum sp., dadurch gekennzeichnet, dass man (a) das Pflanzenmaterial zur Gewinnung eines Direkt-Extraktes, der Kavapyrone als Wertstoffe enthält, einer Hochdruckextraktion mit Kohlendioxid unterzieht, (b) die als Verunreinigungen vorhandenen Flavokavine im Zuge der Hochdruckextraktion durch Behandlung mit teilchenförmigem γ-Aluminiumoxid praktisch vollständig entfernt und (c) das Kohlendioxid vom Direkt-Extrakt trennt.

2. Verfahren nach Anspruch 1, dadurch gekennzeichnet, dass für die Hochdruckextraktion Kohlendioxid in flüssigem unterkritischem Zustand verwendet wird.

3. Verfahren nach Anspruch 1, dadurch gekennzeichnet, dass für die Hochdruckextraktion Kohlendioxid in überkritischem Zustand verwendet wird.

4. Verfahren nach einem der Ansprüche 1 - 3, dadurch gekennzeichnet, dass die Hochdruckextraktion bei einem Druck von 100 - 400 bar und einer Temperatur von 20 - 80°C durchgeführt wird.

5. Verfahren nach einem der Ansprüche 1 - 4, dadurch gekennzeichnet, dass die Dauer der Hochdruckextraktion 10 - 60 Minuten beträgt.

6. Verfahren nach einem der Ansprüche 1 - 5, dadurch gekennzeichnet, dass das Pflanzenmaterial eine Korngrösse von weniger als 500 µm, vorzugsweise eine solche im Bereich von 100 - 350 µm und insbesondere von 125 - 250 µm hat.

7. Verfahren nach einem der Ansprüche 1 - 6, dadurch gekennzeichnet, dass das γ-Aluminiumoxid einen Wassergehalt von höchstens etwa 10 Gew.% hat.

8. Verfahren nach einem der Ansprüche 1 - 7, dadurch gekennzeichnet, dass die Hochdruckextraktion mit einem Lösungsmittelstrom von mindestens etwa 2 kg/Stunde, bezogen auf 100 g Pflanzenmaterial, durchgeführt wird.

9. Vorrichtung zur Durchführung des Verfahrens nach einem der Ansprüche 1 - 8 mit mindestens einem druckfesten Extraktionsbehälter, in den Kohlendioxid unter erhöhtem Druck eingeführt und von dem ein Strom von Direkt-Extrakt enthaltendem Kohlendioxid abgezogen werden kann, gekennzeichnet durch mindestens eine Zone zur Behandlung des extrakthaltigen Kohlendioxids mit γ-Aluminium-oxid, wobei die Zone im Extraktionsbehälter liegt oder/und diesem in einem separaten druckfesten Behälter nachgeschaltet ist.

10. Kawa-Kawa-Extrakt, hergestellt nach dem Verfahren gemäss einem der Ansprüche 1 -9.

11. Kawa-Kawa-Extrakt, der Kavapyrone als Wertstoffe enthält, mindestens teilweise aus kristallinen Anteilen besteht und praktisch frei von Flavokavinen ist, erhältlich durch ein einstufiges Verfahren zur Extraktion von Pflanzenmaterial von Piper methysticum sp.nach einem der Patentansprüche 1 - 9.
